(19) Europäisches Patentamt / European Patent Office / Office européen des brevets

(11) **EP 1 797 887 B1**

(12) **EUROPEAN PATENT SPECIFICATION**

(45) Date of publication and mention of the grant of the patent:
**15.04.2015 Bulletin 2015/16**

(21) Application number: **05790624.0**

(22) Date of filing: **07.10.2005**

(51) Int Cl.:
*A23L 1/308* (2006.01)     *A61K 31/718* (2006.01)
*A61P 3/06* (2006.01)

(86) International application number:
**PCT/JP2005/018610**

(87) International publication number:
**WO 2006/041021 (20.04.2006 Gazette 2006/16)**

(54) **DRUG FOR DIMINISHING INCREASE OF NEUTRAL FAT IN BLOOD AFTER MEAL**

ARZNEIMITTEL ZUR VERRINGERUNG DER ZUNAHME VON NEUTRALEM FETT IM BLUT NACH EINER MAHLZEIT

MÉDICAMENT PERMETTANT DE RÉDUIRE L AUGMENTATION DE LA TENEUR EN GRAISSES NEUTRES DANS LE SANG ÂPRÈS UN REPAS

(84) Designated Contracting States:
**AT BE BG CH CY CZ DE DK EE ES FI FR GB GR HU IE IS IT LI LT LU LV MC NL PL PT RO SE SI SK TR**

(30) Priority: **08.10.2004 JP 2004296310**

(43) Date of publication of application:
**20.06.2007 Bulletin 2007/25**

(73) Proprietor: **MATSUTANI CHEMICAL INDUSTRY CO., LTD.**
**Hyogo, Itami-shi 664-8508 (JP)**

(72) Inventors:
• **KISHIMOTO, Yuka**
**6691537 (JP)**

• **OGA, Hiroshi**
**6660024 (JP)**

(74) Representative: **Hart-Davis, Jason et al**
**Cabinet Beau de Loménie**
**158, rue de l'Université**
**75340 Paris Cedex 07 (FR)**

(56) References cited:
**EP-A- 0 444 891     EP-A- 0 535 627**
**EP-A- 0 616 776     JP-A- 5 255 402**
**JP-A- 6 032 802     JP-A- 6 080 701**
**JP-A- 6 166 622     JP-A- 11 263 733**
**JP-A- 2001 252 064     JP-A- 2003 002 836**
**US-A- 5 358 729     US-A1- 2003 211 201**

**Description**

**Technical Field:**

The present invention relates to an agent for the inhibition of a possible postprandial increase in blood neutral fat level and, more particularly, to an agent for the inhibition of a possible increase in blood neutral fat level, which is ingested together with a high fat-content meal.

**Background Art:**

The life-style related disease is, as the name indicates, one caused by the life-style of each particular subject and the eating habit and the exercise habit thereof would principally be considered to be strongly involved in the life-style related disease. In particular, the changes in the eating habits along with the westernization or Americanization of the eating culture of Japan would be a main cause of the life-style related disease. One of the most characteristic properties of such change in eating habits is that the rate of fat's energy in the total energy intake increases, while the intake rate of the carbohydrates such as cereals is correspondingly decreased (according to the national nutrition survey carried out on 2001). It has been pointed out that the excess intake of fats would not only become a cause of the obesity, but also increases the risk of incidence of the so-called life-style related disease such as hyperlipemia, as well as arteriosclerosis and fatty liver associated with the hyperlipemia.

Conventionally, it has in general been known that the risk of incidence of the diseases of circulatory organs increases due to the excess intake of fats, in particular, cholesterols and accordingly, it has been advocated that the cholesterol content in the serum should be controlled. After that, however, it has been recognized or proved that there is a correlation between the neutral fat value in serum and the number of patients died of the coronary artery diseases and as a result, there has been reported that the correlation is strong as compared with that observed between the cholesterol content and the number of patients died of the diseases. Further, there have successively been submitted a number of reports, which support the foregoing conclusion and accordingly, it has been proved that the neutral fat value would be one of a risk factor for arteriosclerosis. According to the recent researches, factors such as the level of the neutral fat value increased after the intake of a meal and the time required for the reduction thereof to a normal level have attracted special interest lately as risk factors for arteriosclerosis, in addition to the neutral fat value observed when a subject is hungry.

The fats ingested in the form a meal are first digested in the small intestine by the action of the pancreatic lipase and then absorbed and they emerge in the blood in the form of chylomicrons. Accordingly, the neutral fat value increases after the ingestion of a meal. The chylomicrons are then decomposed by the action of a lipoprotein lipase, dissimilated into RLP-cholesterols and then taken by the liver. As a result of such metabolism, the neutral fats originated from a meal would in general be disappeared from the blood after 6 hours from the intake of the meal and returned back to the level corresponding to that observed when the subject is hungry.

When fats are ingested in excess, however, the neutral fat value is greatly raised after the ingestion of the fats and it takes a longer period of time to dissimilate the fats ingested or a longer period of time would be required till the blood fat level is returned back to that observed upon the fasting condition. In this respect, it has been pointed out as follows: it becomes a principal factor for the incidence of arteriosclerosis that the blood fat concentration is maintained at a high level over a long period of time after the intake of a meal and therefore, it has increasingly been desired for the development of any edible material which can reduce the blood level of neutral fats after the intake of a meal.

Incidentally, it has been reported that the repeated ingestion of a hardly digestible dextrin would provide an effect of reducing the total serum cholesterol level in the fasting stomach condition (see, for instance, Patent Document 1 specified below). In addition, it has also been reported that the repeated ingestion of a hardly digestible dextrin would show an effect of reducing the blood level of remnant-like lipoprotein particles-cholesterol (RLP-cholesterol) (see, for instance, Patent Document 2 specified below).

However, these effects are those for reducing, for instance, the serum cholesterol level through the continuous ingestion of such a hardly digestible dextrin, and accordingly they are different from a temporary effect of inhibiting the increase of serum level of neutral fats after eating.

On the other hand, it has likewise been reported that beers and sparkling alcohol-containing beverages, which comprise a hardly digestible dextrin, can inhibit any increase in the serum level of neutral fats, the serum insulin level and/or the blood sugar level after the ingestion of a meal (see Patent Document 3 specified below).

However, this article never refers to the effect of such a hardly digestible dextrin on the high fat-content meal and this article only specifically discloses the effect of inhibiting any increase of the blood sugar level due to the ingestion of a food mainly comprising carbohydrates.

Patent Document 1: EP 0 444 991 A

Patent Document 2: Japanese Un-Examined Patent Publication 2008-2836;
Patent Document 3: Japanese Un-Examined Patent Publication 2001-252064

According to US 2003/0211201 A1, the constant presence of soluble fiber in the digestive tract provides the known beneficial effects of moderating the postprandial increase in blood glucose, modulating serum lipid levels, and suppressing appetite.

EP 0616776 A2 shows a feed for domestic fowls which comprises an indigestible dextrin to inhibit the formation of fatty liver, to improve the meat quality and the rate of egg laying, to improve the eggshell strength and to reduce the cholesterol content in the egg.

US 5,358,729, EP 0 535 627 A1, JP 6 080 701 A, JP 5 255 402 A and JP 6 032 802 A show indigestible fibers such as dextrin to improve the serum composition with respect to lipids, and JP 6 166 662 A teaches the effect of such fibers for inhibiting a postprandial temporary increase in blood sugar when a high sugar meal is ingested.

**Disclosure of the Invention:**

**Problems to be Solved by the Invention:**

The present invention relates to an agent for inhibiting the postprandial increase in blood neutral fat level, when it is ingested together with a high fat-content meal.

**Means for Solving the Problems:**

The inventors of this invention have conducted intensive studies to solve the foregoing problems, have found that the postprandial increase in blood neutral fat level can be inhibited by the ingestion of a water-soluble and hardly digestible starch decomposed product having a content of hardly digestible components of at least 35% by mass, and have thus completed the present invention.

The invention is defined in the claims

**Effects of the Invention:**

The present invention can substantially inhibit any increase in blood neutral fat level possibly encountered when a subject ingests a high fat-content meal and likewise immediately reduce the blood neutral fat level even when one temporarily ingests a high fat-content meal in excess. Therefore, the present invention is quite effective for the prevention of the occurrence of obesity, and any life-style related disease such as hyperlipemia, hypertension and arteriosclerosis.

**Best Mode for Carrying Out the Invention:**

The present agent for inhibiting the postprandial increase in blood neutral fat level or the food according to the present invention comprises a water-soluble and hardly digestible starch decomposed product having a content of hardly digestible components of at least 35% by mass. Such a water-soluble and hardly digestible starch decomposed product is roasted dextrin obtained by decomposing starch through heating in the presence or absence of an acid; hydrolyzates thereof, or hydrogenated products thereof. In this respect, however, when these water-soluble and hardly digestible starch decomposed products contain digestible components, the digestible components are completely or partially removed from these decomposed products to thus increase the content of the hardly digestible components. The content of the hardly digestible components present in the water-soluble and hardly digestible starch decomposed products used in the present invention is not less than 35% by mass, preferably not less than 60% by mass and more preferably not less than 85% by mass as expressed in terms of the solid content thereof. The upper limit thereof is, for instance, particularly preferably 100% by mass. In addition, it is suitable in the present invention that the number average molecular weight of each hardly digestible component ranges : from 500 to 7000, preferably 1000 to 4000.

Preparation of Water-Soluble and Hardly Digestible Components

Starch materials used for the preparation of water-soluble and hardly digestible starch decomposed products are not restricted to particular ones and examples thereof suitably used in the present invention include corn starch, waxy corn starch, potato starch, tapioca starch, sweet potato starch, sago palm starch, wheat starch, barley starch, and rice starch.

First, such a starch material is treated with a mineral acid. Examples of such mineral acids suitably used herein are hydrochloric acid, nitric acid and sulfuric acid. The mineral acid preferably used is hydrochloric acid. The mineral acid is suitably added to the starch material in the form of an aqueous solution thereof and in an amount, for instance, ranging from 3 to 10 parts by mass relative to 100 parts by mass of the starch. The aqueous solution of such a mineral acid suitably used herein has a concentration ranging from 0.5 to 3.0% by mass and preferably 1.0 to 2.0% by mass.

The roasted dextrin can be obtained by heating a starch material in a medium consisting of the foregoing aqueous mineral acid solution. For instance, a starch material is uniformly mixed with such an aqueous mineral acid solution and then the resulting mixture is heated.

The heating operation is desirably carried out in a proper mixer. In this case, the heat treatment is implemented with stirring. The mixture may be aged by the continuation of the heating and stirring operations over 1 to 12 hours.

When heating the mixture, it is preferred that the mixture is preliminarily blended while heating the same at a temperature ranging from 100 to 120°C to thus reduce the water content of the mixture down to a level on the order of 5% by mass. In this connection, it is desirable to implement the heating operation preferably at a temperature ranging from 140 to 200°C for a period of time ranging from 0.2 to 120 minutes and preferably 20 to 120 minutes. The heating operation is preferably carried out at a higher temperature since the higher the temperature used in the heating operation, the higher the content of the hardly digestible components in the resulting heat-treated product. In this respect, however, the content of colored substances present therein begins to increase as the temperature is getting near to 180°C and accordingly, the heating operation is preferably carried out at a temperature, for instance, around 150°C

It is also possible to cause reaction at a high temperature for a short period of time, by appropriately selecting a heating device. Accordingly, such a mixture can efficiently be subjected to a heating treatment when using a device such as an extruder which permits the implementation of a uniform reaction at a high temperature for a short period of time. In addition, the starch material is used in its powdery state in the foregoing reaction and the heating conditions should be changed in case of a large-scale production of roasted starch. Therefore, it is desirable that the heating conditions are appropriately changed while strictly taking into consideration the quality of the products obtained after such heat-treatment.

The roasted dextrin product thus prepared can be used as a water-soluble and hardly digestible starch decomposed product after it is treated with, for instance, a strongly acidic ion-exchange resin for the removal of the digestible components, without subjecting the same to any further treatment. The content of the hardly digestible components in the roasted dextrin product from which the digestible components are removed ranges from, for instance, 50 to 65% by mass.

In this connection, the digestible component is almost or mainly composed of low molecular weight digestible components derived from glucose.

The roasted dextrin can further be subjected to hydrolysis using, for instance, an enzyme or an acid to thus convert it into hydrolyzates having smaller molecular weights, optionally followed by the removal of the digestible components from the hydrolyzates thus obtained to thus give a composition having a high content of hardly digestible components.

For instance, the roasted dextrin thus prepared is dissolved in water in a concentration ranging, for instance, from 20 to 50% by mass, the pH value of the resulting solution is adjusted to a level ranging, for instance, from 5.5 to 6.5, preferably 6.0 using, for instance, a neutralizer such as sodium hydroxide, a liquefying type α-amylase is added to the solution in an amount, for instance, ranging from 0.05 to 0.2% by mass, the solution is hydrolyzed at a temperature ranging from 80 to 95°C, which corresponds to the working temperature of α-amylase, for a time in general about one hour and then the temperature of the reaction system is increased up to 120°C to thus deactivate the enzyme or the α amylase. This liquefying type α-amylase may be any commercially available one and a specific example thereof includes Termamyl 120L (the trade name of a product available from Novozyme Japan).

Then, the temperature of the solution is first reduced to 60°C, the pH value thereof is adjusted to a level ranging from, for instance, 4 to 5, preferably 4.5, glucoamylase is added to the solution in an amount ranging from 0.05 to 0.4% by mass, the hydrolysis of the solution is carried out at a temperature ranging from, for instance, 55 to 60°C, over a time ranging from, for instance, 4 to 48 hours to thus convert the components other than the hardly digestible ones into glucose, and the temperature of the reaction system is then raised up to 80°C to thus deactivate the enzyme or the glucoamylase. The glucoamylase of this type may be any commercially available one and a specific example thereof includes Gluczyme NL4.2 (the trade name of a product commercially available from Amano Enzyme Co., Ltd.). After that, the product thus obtained may further be subjected to the currently used decoloration treatment with activated charcoal, filtration, and desalting and decoloring with an ion-exchange resin to thus concentrate the same to a concentration on the order of 50% by mass.

Alternatively, can be used a method which comprises the step of hydrolysis in the presence of an acid, in addition to the foregoing hydrolysis method which makes use of an enzyme. The roasted dextrin is easily soluble in water and accordingly, water can be added thereto with stirring to thus give an aqueous solution thereof. The resulting aqueous solution may then be hydrolyzed without any additional treatment or after the pH value thereof is adjusted to a level of, for instance, 1.6 to 2.0 by the addition of an acid such as hydrochloric acid or oxalic acid. The hydrolysis is carried out by heating the roasted dextrin, under pressure, at a temperature ranging from, for instance, 120 to 140°C for a time ranging from, for instance, 15 to 30 minutes. The acid-hydrolyzate thus prepared may further be subjected to the currently used decoloration treatment with activated charcoal, filtration, and desalting and decoloring with an ion-exchange resin to thus concentrate the same to a concentration on the order of 50% by mass.

The roasted dextrin and hydrolyzate thereof thus obtained can then be passed through a column packed with a strongly acidic cation-exchange resin to thus fractionate them into the hardly digestible components and the digestible components according to the chromatographic separation system and to thus give a product having a content of the hardly digestible components of 85 to 95% on the basis of the total mass of the solid contents thereof.

Incidentally, the hardly digestible component contains glucose as the constituent thereof and includes 1→2 and 1→3 bonds in addition to the 1→4 and 1→6 bonds. Moreover, when reducing the hardly digestible component, the portion of the reducing ends is converted into 1→6 anhydroglucose moieties.

The foregoing strongly acidic cation-exchange resins used in the separation of the hardly digestible components from the digestible components may be a various kinds of commercially available ones. Specific examples thereof preferably used herein include Amberlite IR-116, Amberlite IR-118, Amberlite IR-120B, XT-1022E and XT-471F (the trade names of products available from ORGANO Company); DIAION SK-1B, DIAION SK102, DIAION SK104, DIAION SK106, DIAION SK110, DIAION SK112, DIAION SK116, and DIAION FR01 (the trade names of products available from Mitsubishi Chemical Industries Ltd.); and XFS-43281.00, XFS-43280.00, XFS-43279.00, and XFS-43278.00 (the trade names of products available from Dow Chemical Japan Co., Ltd.).

It is preferred that these resins are converted into their alkali metal-forms or alkaline earth metal-forms before they are put into practical use. The flow rate (the space velocity: SV) of the roasted dextrin and hydrolyzate thereof prepared above which are passed through the column packed with the ion-exchange resin preferably ranges from 0.1 to 0.6. In this respect, if the flow rate thereof is beyond the foregoing range, the working properties and the separation ability of the resin are liable to be impaired or insufficient. The temperature of the solution when passing the same through the column preferably ranges, for instance, from 20 to 70°C. This is because if it is less than the lower limit, the solution is insufficiently fractionated, the viscosity of the solution increases and accordingly, the resin may be damaged, while if it exceeds the upper limit, the quality of the resulting liquid is reduced due to the browning and the resin may possibly be deteriorated.

The water-soluble and hardly digestible starch decomposed product according to the present invention may be one obtained by further subjecting the foregoing roasted dextrin or the hydrolyzate thereof to hydrogenation. This hydrogenation (reducing) reaction can be carried out under the conditions identical to those currently used for the treatment of starch and sugar. The reducing reaction is suitably carried out through hydrogenation in the presence of a currently used reducing catalyst such as Raney nickel catalyst, Raney cobalt catalyst or nickel-diatomaceous earth catalyst, at a hydrogen pressure ranging, for instance, from 50 to 130 kg/cm$^2$ and a temperature ranging, for instance, from 50 to 150°C. The heating operation during the reducing step is preferably carried out after hydrogen is sufficiently dissolved in the solution till the solution is completely saturated therewith, while if the hydrogen is insufficiently supplied, this reducing reaction may sometimes be accompanied by undesirable side reactions such as oxidation and/or hydrolysis. This hydrogenation is usually completed within 2 hours although the hydrogenation time may vary, to a certain extent, depending on the reaction conditions such as the temperature and pressure conditions selected. Thereafter, the resulting solution may be purified according to the usual purification techniques such as the additional decoloration with activated charcoal after the removal of the catalyst, filtration, and/or desalting and decoloring with an ion-exchange resin, followed by the concentration of the purified solution and the subsequent drying thereof to thus give a powdery product, or the resulting solution may be subjected to a finishing concentration treatment to a final concentration on the order of 70% by mass to thus give a liquid product.

The water-soluble and hardly digestible starch decomposed product suitably used in the present invention may be commercially available ones and specific examples thereof commercially available include "Pinefiber Bi", "Fibersol 2", hydrogenated type ones such as "Fibersol 2H" and "Fibersol 2HL" (liquid products) (they are all available from Matsutani Chemical Industry Co., Ltd.) and Nutriose (available from Roquette Co., Ltd.).

The high fat-content meals used in the present invention are foods rich in fat components. The term "food rich in fat components" herein used means a food having a fat-content ranging from 20 to 30% by mass.

The techniques for determining the fat-content have been well known to one of ordinary skill in the art. The fat-content may easily be determined according to, for instance, the Soxhlet's extraction technique.

The high fat-content meals suitably used along with the water-soluble and hardly digestible starch decomposed product may be, for instance, fats in themselves such as beef tallow, hog fat, chicken fat, and vegetable fats; and foods each having a high fat-content such as ice cream, hamburg, sausage, butter, and chocolate. The high fat-content meal may be in the form of a solid or semi-solid.

In the present invention, the water-soluble and hardly digestible starch decomposed product is incorporated into a high fat-content meal.

The decomposed product is suitably used in such a manner that the ratio, by mass, of the amount of fats present in a high fat-content meal to be ingested to that of the hardly digestible components present in the water-soluble and hardly digestible starch decomposed product falls within the range of from 1:0.05 to 1:0.50 and preferably 1:0.08 to 1:0.40 (mass ratio).

The present invention will hereunder be described in mode detail with reference to the following Examples.

**EXAMPLES:**

<Method for Quantitative Analysis of Hardly Digestible Components>

The hardly digestible components present in the water-soluble and hardly digestible starch decomposed product can quantitatively be analyzed according to the high performance liquid chromatography technique (enzyme-HPLC technique) which is a technique for analyzing edible fibers as disclosed in EISHIN No. 13 (relating to methods or the like for analyzing, for instance, nutrient components in Nutrient-Denotation Standard).

<Determination of Number Average Molecular Weight>

The number average molecular weights of the hardly digestible components are determined using the high performance liquid chromatography technique under the following conditions:

Column Used: TSKgel G2500PWXL, G3000PWXL, G6000PWXL (available from Tosoh Corporation);
Detector: Differential Refractometer;
Column Temperature: 80°C;
Flow Rate: 0.5 mL/min;
Mobile Phase: Distilled water;
Amount of Sample Used: 100 $\mu$l (1% by mass solution).

The number average molecular weight was calculated using the calibration curve determined using Multi-Station GPC-8020 (available from Tosoh Corporation), the pullulan reference substance (having a known molecular weight) as well as malto-triose and glucose, according to the following equation:

$$Mn = \Sigma\, Hi / \Sigma\, (Hi/Mi) \times QF$$

Mn: number average molecular weight of each particular sample
Hi: peak height
Mi: the molecular weight of pullulan
QF: Q factor (Mark-Houwink's coefficient)

**Example 1**

Rats were used as test animals and fat-loading tests were carried out using the samples specified in the following Table 1. First, 10-week-old SD male rats (each group consisted of 5 animals) were fasted overnight, they were divided into the following groups and samples were administered to respective groups:

Control Group (only corn oil (1 g/kg) was administered);
Composition A-Administered Group (1 g/kg of corn oil + 1 g/kg of Sample A);
Composition B-Administered Group (1 g/kg of corn oil + 1 g/kg of Sample B);
Composition C-Administered Group (1 g/kg of corn oil + 1 g/kg of Sample C);
Composition D-Administered Group (1 g/kg of corn oil + 1 g/kg of Sample D); and
Composition E-Administered Group (1 g/kg of corn oil + 1 g/kg of Sample E).

Blood samples of each animal were collected before the administration of the fat and/or each sample and after 1, 2, 3, 4, 5, 6 and 7 hours from the administration thereof through the caudal vein and the resulting blood plasma was used for the determination of the blood level of neutral fats.

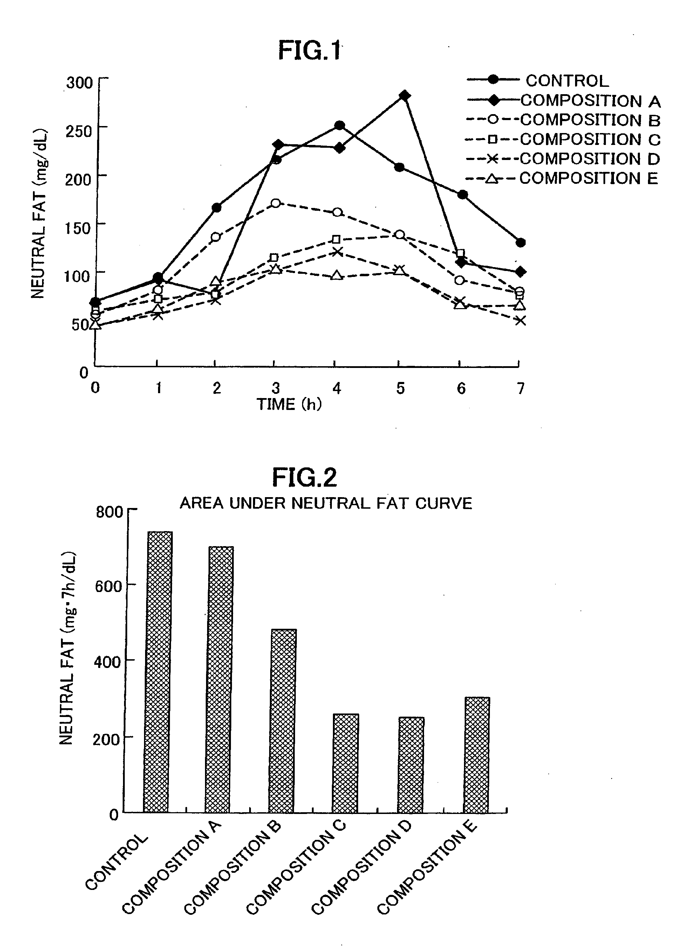

The neutral fat value observed for the control animals, to which only corn oil had been administered, showed such a pattern that the value gradually increased with time after the loading thereof with the fat, reached the highest level after 4 hours and then gradually reduced (see Fig. 1).

The composition A-administered group, to which the composition A free of any hardly digestible component had been administered, showed the neutral fat values higher than those observed for the control group after 3 and 5 hours from the administration of the composition A. Then, the area under each curve showing the change of neutral fats with time (hereunder referred to as "AUC") was calculated for these curves and the resulting areas were compared with one another and as a result, it was found that these areas were almost identical to one another (see Fig. 2). On the other hand, it was found that the neutral fat value was low as compared with that observed for the control group and that AUC was likewise lower than that observed for the control group, in all of the test groups, or compositions B, C, D and E-administered groups wherein the compositions B, C, D and E each comprised at least 35% by mass of hardly digestible components, as expressed in terms of the amount converted into the solid content. When comparing the AUC values observed for the compositions B, C, D and E-administered groups with each other, the composition B-administered group in which the composition B had the lowest content of the hardly digestible components was found to have the highest AUC value, while the compositions C and D-administered groups in which the compositions C and D had the same content of the hardly digestible components on the order of 90% were found to have the lowest AUC values which were almost identical to one another. In addition, the composition E-administered group in which the composition E had a hardly digestible component content of 85% showed the AUC value slightly higher than those observed for the compositions C and D-administered groups. These results clearly indicate that the composition containing the hardly digestible components has an effect of inhibiting any increase in the neutral fat level after the administration thereof and that the effect thereof is dependent upon the content of the hardly digestible components (see Fig. 2).

**Table 1**

| Sample | Component | Content of HDC* (% by mass) | Mn** |
|---|---|---|---|
| A (Cont.) | Malto-Dextrin (Trade Name: TK-16, available from Matsutani Chemical Industry Co., Ltd.) | 0 | 900 |
| B | Branched corn syrup (Trade Name: Pinefiber Bi available from Matsutani Chemical Industry Co., Ltd.) | 35 | 600 |
| C | Hardly digestible dextrin (Trade Name: Fibersol 2, available from Matsutani Chemical Industry Co., Ltd.) | 90 | 2000 |
| D | Reduced hardly digestible dextrin (Trade Name: Fibersol 2H, available from Matsutani Chemical Industry Co., Ltd.) | 90 | 2000 |
| E | Edible fiber-enriched dextrin (Trade Name: Neutriose FB, available from Roquette Japan Co., Ltd.) | 85 | 6000 |
| *: Hardly digestible components; **: Average molecular weight. | | | |

**Reference Example 2**

Meal-loading tests were carried out while using 17 subjects consisting of healthy adult men and women. They were fasted since 21:00 of the day before the beginning of the test, while they could freely take water and they took respective test meals in the early morning upon the fasting condition. Blood samples (about 10 ml each) of each subject were collected before the ingestion of each test meal and after 1, 2, 3, 4, 5 and 6 hours from the ingestion thereof through the cubital vein and each blood sample was inspected for the contents of neutral fats and RLP-cholesterol. The test meals or diets were as follows: a hamburger, about 140 g of fried potato; and 340 ml of a carbonated beverage, whose nutritive values were found to be 966 kcal as amount of heat; 34 g of proteins; 49.5 g of fats; and 96 g of carbohydrates. Hardly digestible dextrin (Trade name: Fibersol 2, available from Matsutani Chemical Industry Co., Ltd.) containing 90% by mass of hardly digestible components was used as the substance to be tested and 5 g of the substance (4.5 g as expressed in terms of the hardly digestible component) was added to the carbonated beverage, among other test diets and the test subjects ingested the resulting beverage. Each meal-loading test was repeated twice for each subject, or each subject was once subjected to a test in which he took a placebo beverage free of any test substance (control test) and once to another test in which he took the test substance-containing beverage. The order of these different loading tests was random and it was kept secret for the test subject.

The resulting test results each were expressed in terms of [the average value] ± [standard deviation], analyzed according to the t-test showing the correspondence and the case showing a risk rate of not more than 5% was judged to be significant according to the two-sided test.

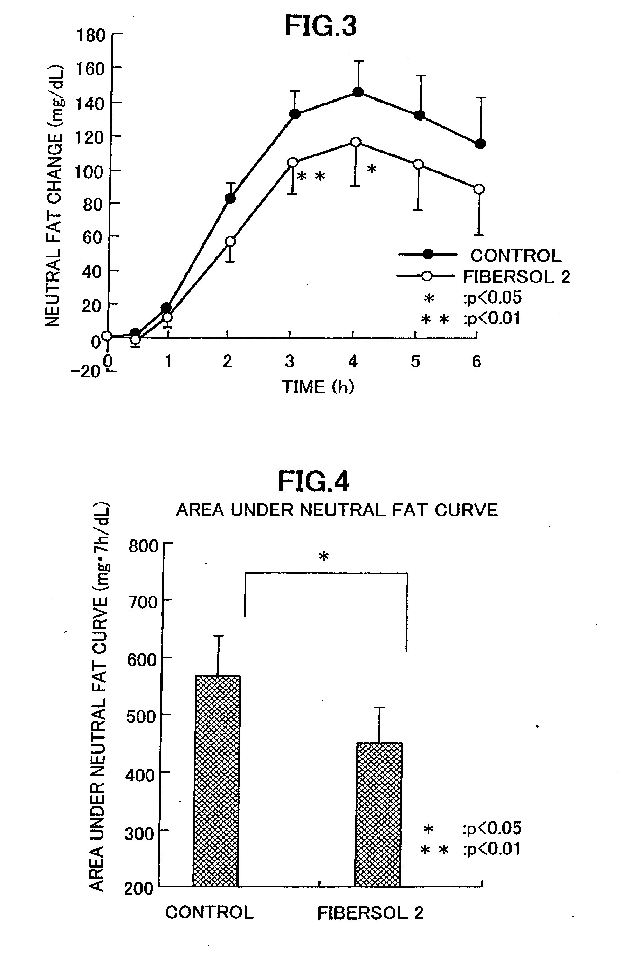

Fig. 3 shows the change of the neutral fat value with the elapse of time observed after the ingestion of the diet, while Fig. 4 shows the area under each curve showing the change of neutral fats with time (AUC).

As a result, it was confirmed that any rise of the neutral fat level after the ingestion of a diet could be controlled by the ingestion of the water-soluble and hardly digestible starch decomposed product having a content of hardly digestible components of 90%.

### reference Example 3

A carbonated beverage (for 5 diets) was prepared according to the formulation specified in the following Table 2:

**Table 2**

| Raw Material | Amt. Incorporated (g) |
|---|---|
| Fibersol 2H | 50 |
| Granulated sugar | 125 |
| Citric acid | 1.5 |
| Sodium citrate | 0.5 |
| Vitamin C | 0.15 |
| Cider essence | 1 |
| Carbonated water | 520 |
| Water | 885 |

This carbonated beverage included 4.1% by mass of the hardly digestible components originated from Fibersol 2H. When one ingested this carbonated beverage, after the ingestion of a high fat-content diet, the blood level of the neutral fats after the ingestion of such a diet could be reduced as in the case described in Example 1 or reference Example 2. In this respect, the mass ratio of the amount of the fats present in the diet to the mass of the hardly digestible components was found to be 1:0.18.

### Example 4

According to the formulation specified in the following Table 3, bitter chocolate and cacao butter were melted and the remaining ingredients other than lecithin were incorporated or kneaded into the resulting melt, followed by the refining of the mixture with a roller mill, the addition of lecithin, and the subsequent tempering of the resulting mixture to thus give chocolate (for 2 to 3 diets).

**Table 8**

| Raw Material | Amt. Incorporated (g) |
|---|---|
| Powdered sugar | 35 |
| Fibersol 2 | 15 |
| Bitter chocolate | 20 |
| Cacao butter | 15 |
| Powdered milk | 14.7 |
| Lecithin | 0.3 |

The resulting chocolate had a fat content of 29.8% by mass, Fibersol 2 comprised 90% by mass of hardly digestible components and thus the mass ratio of the amount of the fats present in the chocolate to the mass of the hardly digestible components was found to be 1:0.45. When one ingested this chocolate, the blood neutral fat level after the ingestion of a diet could be reduced as in the case described in Example 1 or reference Example 2.

### reference Example 5

According to the formulation specified in the following Table 4, all of the ingredients were mixed together, followed by a heat-treatment at 80°C for the melting thereof, homogenization of the molten mixture, the subsequent aging over 24 hours and cooling of the mixture at a temperature of -40°C to thus give ice cream (for one diet).

**Table 4**

| Raw Material | Amt. Incorporated (g) |
|---|---|
| Raw cream | 8.4 |
| Butter | 4.2 |
| Sweetened condensed milk | 19.2 |
| Powdery skimmed milk | 2.3 |
| Sugar | 2.5 |
| Fibersol 2H | 5 |
| Emulsion-stabilizing agent | 0.7 |
| Vanilla flavor | 0.1 |
| Water | 57.6 |

The resulting ice cream had a fat content of 8.9% by mass, Fibersol 2H comprised 90% by mass of hardly digestible components and thus the mass ratio of the amount of the fats present in the ice cream to the mass of the hardly digestible components was found to be 1:0.50. When one ingested this ice cream, the blood neutral fat level after the ingestion of the diet could be reduced as in the case described in Example 1 or reference Example 2.

## Example 6

According to the formulation specified in the following Table 5, cooked rice was prepared. The mass of the raw materials was found to be 295.5 g prior to the cooking, but it was reduced to 264.59 g after the cooking and thus the content of the reduced hardly digestible dextrin per 180 g (for one diet) of the cooked rice was found to be equal to 5.102 g (2.83% by mass) (the content of the hardly digestible components was found to be 4.59 g (2.55% by mass)).

**Table 5**

| Raw Material | Amt. Incorporated (g) |
|---|---|
| Water-immersed rice (100 g of raw rice was immersed in water for 20 minutes) | 128 |
| Fibersol 2H | 7.5 |

This cooked rice comprised 2.55% by mass of the hardly digestible components derived from Fibersol 2H. When one ingested this cooked rice, the blood neutral fat level after the ingestion of such a diet could be reduced as in the cases described in Example 1 or reference Example 2. In this connection, the mass ratio of the amount of the fats present in the cooked rice to the mass of the hardly digestible components was found to be 1:0.09.

## Example 7

According to the formulation specified in the following Table 6, the raw materials were mixed in a Kenmix mixer, formed into a desired shape and then baked at 170°C for 3 minutes for each side to thus give hamburg (for one diet).

**Table 6**

| Raw Material | Amt. Incorporated (g) |
|---|---|
| Minced pork | 21.1 |
| Minced beef | 19.0 |
| Minced chicken | 20.0 |
| Common salt | 0.5 |
| Sugar | 0.7 |

(continued)

| Raw Material | Amt. Incorporated (g) |
|---|---|
| Sodium glutamate | 0.5 |
| White pepper | 0.1 |
| Nutmeg | 0.1 |
| Hog fat (lard) | 20.0 |
| Onion | 10.0 |
| Whole egg | 3.0 |
| Starch | 5.0 |
| Fibersol 2 | 6.3 |

This hamburg had a fat content of 26.3% by mass and contained 5.3% by mass of the hardly digestible components derived from Fibersol 2 and accordingly, the mass ratio of the amount of the fats present in the hamburg to the mass of the hardly digestible components was found to be 1:0.20. When one ingested this hamburg, the blood neutral fat level after the ingestion of the diet could be reduced as in the case described in Example 1 or reference Example 2.

### Example 8

According to the formulation specified in the following Table 7, all of the raw materials were blended together, the resulting mixture was deaerated, charged into naturally occurring gut-casings, salted at 5°C for 20 hours, followed by drying and smoking at 40°C for 80 minutes, boiling with steam and the subsequent cooling down to 5°C to thus give Vienna sausage (for 2 diets).

**Table 7**

| Raw Material | Amt. Incorporated (g) |
|---|---|
| Minced lean pork | 40.0 |
| Hog fat | 20.0 |
| Starch | 5.0 |
| Common salt | 1.0 |
| Sugar | 1.0 |
| Casein sodium | 0.5 |
| Sodium glutamate | 0.5 |
| Spices | 0.5 |
| Sodium tripolyphosphate | 0.2 |
| L-Ascorbic acid | 0.05 |
| Sodium nitrite | 0.006 |
| Fibersol 2 | 10.0 |
| Water | 20.944 |

The resulting Vienna sausage had a fat content of 22.0% by mass, likewise contained 9.0% by mass of the hardly digestible components derived from Fibersol 2 and therefore, the mass ratio of the amount of the fats present in the sausage to the mass of the hardly digestible components contained therein was found to be 1:0.40. When one ingested this Vienna sausage, the blood neutral fat level after the ingestion of the diet could be reduced as in the case described in Example 1 or reference Example 2.

### reference Example 9

According to the formulation specified in the following Table 8, a tea beverage (for one diet) was prepared.

**Table 8**

| Raw Material | Amt. Incorporated (g) |
|---|---|
| Vitamin C | 0.05 |
| Mixed tea leaves | 95.55 |
| Fibersol 2H | 4.4 |

The resulting tea beverage comprised 3.96% by mass of the hardly digestible components originated from Fibersol 2H and accordingly, when ingesting this tea beverage after one ingested the high fat-content meal disclosed in Example 2, the blood neutral fat level after the ingestion of the meal could be reduced as in the case described in Example 1 or reference Example 2. In this connection, the mass ratio of the fats contained in the high fat-content meal to the hardly digestible components ingested was found to be 1:0.08.

**Brief Description of the Drawings :**

Fig. 1 is a graph showing the changes of the neutral fat level in the blood as a function of time, observed when administering a variety of compositions and corn oil to rats, for the purpose of the comparison.

Fig. 2 is a bar graph showing the calculated areas under the respective curves (AUC) shown in Fig. 1, for the purpose of the comparison.

Fig. 3 is a graph showing the changes, with time, in blood neutral fat levels observed when carrying out meal-loading tests (reference Example 2) using healthy adult persons as test subjects and a meal containing the composition (Fibersol 2), wherein the changes are shown on this figure, while comparing the same with those observed for the control.

Fig. 4 is a bar graph showing the calculated areas under the respective curves (AUC) shown in Fig. 3, for the purpose of the comparison.

**Claims**

1. An agent

   - for use in the prevention of obesity, hyperlipemia, hypertension and arteriosclerosis by inhibiting a postprandial temporary increase in blood neutral fat levels within a period of 7 hours after ingestion of a high-fat content meal having a fat-content of 20 to 30% by mass,
   - comprising a water-soluble and hardly digestible starch decomposed product which is roasted dextrin having an average molecular weight from 500 to 7,000 and obtained by decomposing starch through heating in the presence of a mineral acid, hydrolyzates or hydrogenated products thereof, and
   - having a content of hardly digestible components of at least 35% by mass as expressed in terms of the solid content of said starch decomposed product and
   - for use by ingesting said agent simultaneously with said high fat-content meal having the fat-content of 20 to 30% by mass, and
   - for use by incorporating said agent into the high fat-content meal, which is solid or semi-solid.

2. The agent of claim 1 for use by ingesting said agent in such a manner that the ratio, by mass, of the content of fats in said high fat-content meal to be ingested to that of said hardly digestible components to be ingested falls within the range of from 1:0.05 to 1:0.50.

**Patentansprüche**

1. Mittel

   - zur Verwendung für die Vorbeugung von Adipositas, Hyperlipidämie, Hypertonie und Arteriosklerose durch Hemmung einer postprandialen temporären Zunahme des Spiegels von neutralem Fett im Blut für eine Dauer von 7 Stunden nach Aufnahme einer fettreichen Mahlzeit, die einen Fettgehalt von 20 bis 30 Massen-% aufweist,
   - umfassend ein wasserlösliches und schwer verdaubares Produkt aus zersetzter Stärke, welches geröstetes Dextrin mit einem mittleren Molekulargewicht von 500 bis 7.000 ist und erhalten ist durch Zersetzung von Stärke durch Erwärmung in Gegenwart einer Mineralsäure, sowie Hydrolysate und hydrogenierte Produkte von diesem, und
   - aufweisend einen Gehalt an schwer verdaubaren Bestandteilen von mindestens 35 Massen-% ausgedrückt als Feststoffanteil des besagten Produkts aus zersetzter Stärke und
   - zur Verwendung durch gleichzeitige Aufnahme des besagten Mittels mit der besagten fettreichen Mahlzeit, die einen Fettgehalt von 20 bis 30 Massen-% aufweist, und
   - zur Verwendung durch Einarbeiten des besagten Mittels in die fettreiche Mahlzeit, die fest oder halbfest ist.

2. Das Mittel nach Anspruch 1 zur Verwendung durch Aufnahme des besagten Mittels derart, dass das Massenverhältnis des Fettgehalts in der besagten aufzunehmenden fettreichen Mahlzeit zu dem der aufzunehmenden schwer verdaubaren Bestandteile in einen Bereich von 1:0,05 bis 1:0,50 fällt.

**Revendications**

1. Un agent

   - pour utilisation dans la prévention de l'obésité, de l'hyperlipémie, de l'hypertension et de l'artériosclérose en inhibant une augmentation temporaire postprandiale des niveaux de graisse neutre dans le sang durant une période de 7 heures après une ingestion de repas à forte teneur en graisse ayant une teneur en graisse entre 0.2 et 0.3 en pourcentage massique,
   - comprenant un produit décomposé d'amidon difficilement digestible et soluble dans l'eau qui est de la dextrine grillée ayant une masse moléculaire moyenne entre 500 et 7000 et obtenue en décomposant de l'amidon par un réchauffement en présence d'un acide minéral, d'hydrolysat ou de produits hydrogénés de celui-ci, et
   - ayant une teneur en éléments difficilement digestibles d'au moins 0.35 en pourcentage massique comme exprimé en termes de teneur solide dudit produit décomposé d'amidon et
   - pour utilisation par ingestion dudit agent simultanément avec ledit repas à forte teneur en graisse ayant une teneur en graisse de 0.2 à 0.3 en pourcentage massique, et
   - pour utilisation par incorporation dudit agent dans le repas à forte teneur en graisse, qui est solide ou semi-solide.

2. L'agent selon la revendication 1 pour utilisation par ingestion dudit agent de telle manière que le ratio, en masse, de la teneur en graisse dans ledit repas à forte teneur en graisse destiné à être ingéré par rapport à celle des éléments difficilement digestibles destinés à être ingérés se situe dans la plage entre 1/0.05 et 1/0.50.

## FIG.1

## FIG.2

AREA UNDER NEUTRAL FAT CURVE

# FIG.3

# FIG.4
## AREA UNDER NEUTRAL FAT CURVE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0444991 A
- JP 2008002836 A
- JP 2001252064 A
- US 20030211201 A1
- EP 0616776 A2
- US 5358729 A
- EP 0535627 A1
- JP 6080701 A
- JP 5255402 A
- JP 6032802 A
- JP 6166662 A